# EUROPEAN PATENT APPLICATION

(11) **EP 4 844 773 A1**
(43) Date of publication of application: **25.11.2026**
(21) Application number: 24918804.6
(22) Date of filing: 16.01.2024
(51) Int. Cl.: G16B 50/00

(54) **GENOME DATA PROCESSING SYSTEM, GENOME DATA PROCESSING METHOD, AND GENOME DATA PROCESSING PROGRAM**

(71) Applicant: NEC Corporation, 108-8001 Tokyo (JP)
(72) Inventor: TANAKA, Yuki, Tokyo 108-8001 (JP); ONOUE, Kousuke, Tokyo 108-8001 (JP); YOSHIHARA, Yoshiko, Tokyo 108-8001 (JP); YOKOTA, Haruki, Tokyo 108-8001 (JP)
(74) Representative: Betten & Resch
(86) International application number: PCT/JP2024/000897
(87) International publication number: WO 2025/154156

(57) **Abstract**

A genomic data processing system comprising a company server responsible for a part of a series of processes for personalized medicine using genomic data obtained from a specimen of a user, and a management apparatus that manages information regarding the series of processes performed on said genomic data, wherein the company server submits a request for processing to the management apparatus prior to executing the processing, and the management apparatus notifies the company server of a result regarding whether the requested processing is permitted or not, based on permission conditions representing a processing authorized by the user.

## Description

### TECHNICAL FIELD

The present invention relates to a genomic data processing system, a method for processing genomic data, and a genomic data processing program.

### BACKGROUND ART

Traditionally, health care has been primarily disease-centered, with the main objectives being to identify the causes of diseases and develop treatments for them. While it has long been recognized that the same treatment is not equally effective for everyone, even for the same disease, this was difficult to address because individual variances in clinical outcomes could only be determined by observing the treatment and its effects, making it challenging to develop treatment plans optimized for each individual.

Meanwhile, advances in technologies such as DNA sequencing and the identification of single-nucleotide polymorphisms (SNPs) within DNA sequences have made it possible to observe how individual genes differ from those of others. Consequently, it has become practicable to use this information to select the most appropriate treatment for a specific patient or to develop and manufacture medicines optimized for an individual. For example, Patent Literature 1 describes a method for obtaining highly reliable genomic variant information from read data obtained from a next-generation sequencer.

### CITATION LIST

### PATENT LITERATURE

[Patent Literature 1]
Japanese Patent Kokai Publication No. JP2017-016665A

### SUMMARY

### TECHNICAL PROBLEM

The disclosures in the above prior art documents are hereby incorporated by reference into this document. The following analyses were conducted by the inventors.

Meanwhile, genomic data representing the nucleotide sequences of DNA obtained from an individual is, in many cases, generally considered to constitute a "personal identification code" and therefore requires strict management as personal information. Furthermore, it is anticipated that in many cases, "genomic information"-which involves applying medical interpretations, such as information on variants, to genomic data obtained from patients-will be treated as sensitive personal information requiring special care in its handling.

Furthermore, since such genomic data is considered personal information, it is not sufficient to merely protect it from leaks or tampering. Specifically, entities that receive genomic data must process it only in accordance with the consent given by the data provider. These entities are required not only to protect the data from leaks or tampering but also to strictly manage the processing they perform on the genomic data.

In view of the aforementioned issues, the objective of the present invention is to provide a genomic data processing system, a genomic data processing method, and a genomic data processing program that contribute to managing a series of processes for personalized medicine based on genomic data obtained from a specimen of a user.

### SOLUTION TO PROBLEM

According to a first aspect of the present invention, there is provided a genomic data processing system comprising a company server responsible for a part of a series of processes for personalized medicine using genomic data obtained from a specimen of a user, and a management apparatus that manages information regarding the series of processes performed on said genomic data, wherein the company server submits a request for processing to the management apparatus prior to executing the processing, and the management apparatus notifies the company server of a result regarding whether the requested processing is permitted or not, based on permission conditions representing a processing authorized by the user.

According to a second aspect of the present invention, there is provided a method for processing genomic data, using a company server responsible for part of a series of processes for personalized medicine based on genomic data obtained from a specimen of a user, and a management apparatus that manages information regarding the series of processes performed on said genomic data, wherein the company server submits a request for processing to the management apparatus prior to executing the processing, and the management apparatus notifies the company server of a result regarding whether the requested processing is permitted or not, based on permission conditions representing a processing authorized by the user.

According to a third aspect of the present invention, there is provided a genomic data processing program that causes a company server responsible for part of a series of processes for personalized medicine based on genomic data obtained from a specimen of a user, and a management apparatus that manages information regarding the series of processes performed on said genomic data, to execute genomic data processing, wherein the genomic data processing program causes the company server submit a request for processing to the management apparatus prior to executing the processing, and the management apparatus notify the company server of a result regarding whether the requested processing is permitted or not, based on permission conditions representing a processing authorized by the user. Further, this program can be stored in a computer-readable storage medium. The storage medium may be a non-transitory one such as a semiconductor memory, a hard disk, a magnetic recording medium, an optical recording medium, and the like. The present invention can also be realized as a computer program product.

### ADVANTAGEOUS EFFECTS OF INVENTION

According to each aspect of the present invention, a genomic data processing system, a genomic data processing method, and a genomic data processing program that contribute to managing a series of processes for personalized medicine based on genomic data obtained from a specimen of a user can be provided.

### BRIEF DESCRIPTION OF THE DRAWINGS

[Fig. 1]

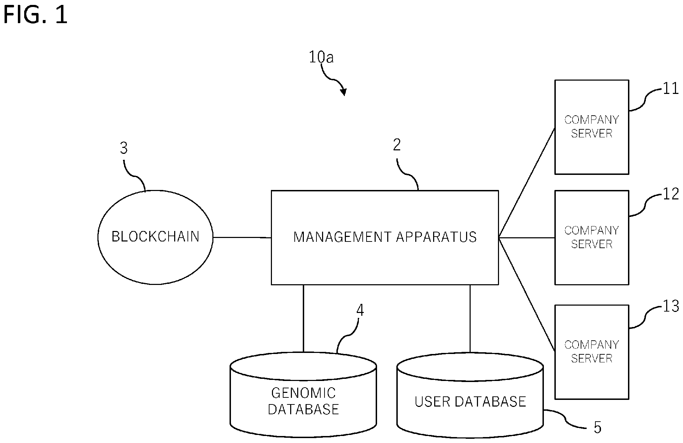

   Fig. 1 is a diagram illustrating an example of the schematic configuration of a genomic data processing system.
[Fig. 2]

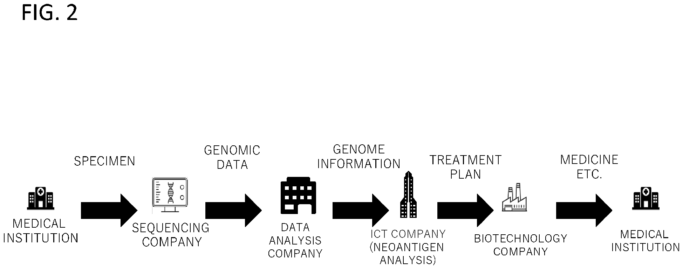

   Fig. 2 is a schematic diagram illustrating an example of the series of processes applied to genomic data in personalized medicine.
[Fig. 3]

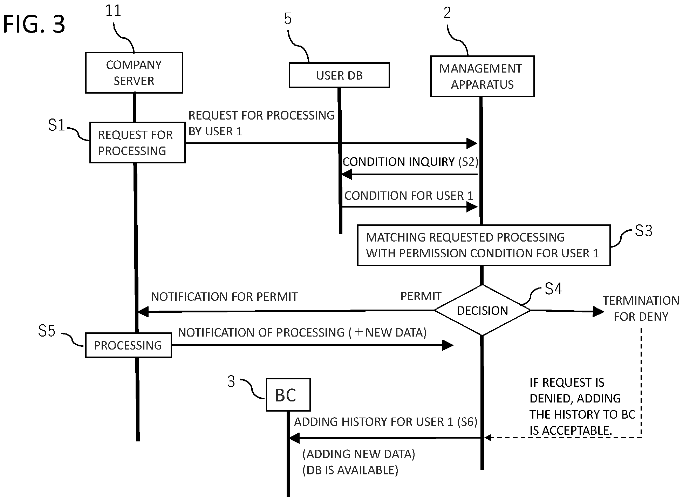

   Fig. 3 illustrates an example of the processing performed for personalized medicine using genomic data obtained from a specimen of a user.
[Fig. 4]

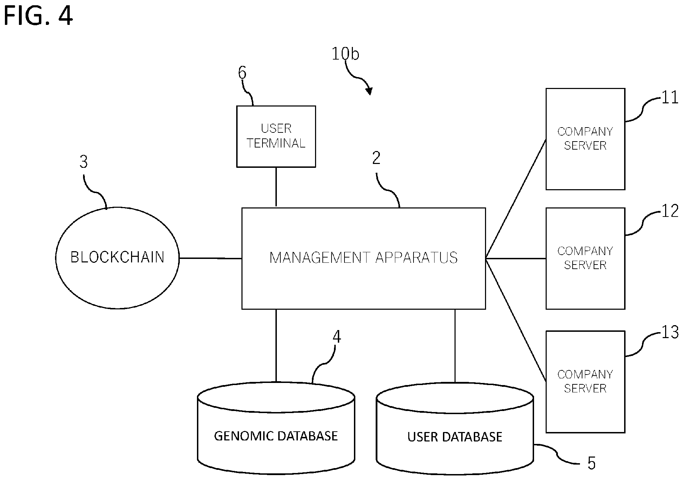

   Fig. 4 is a diagram illustrating an example of the schematic configuration of a genomic data processing system.
[Fig. 5]

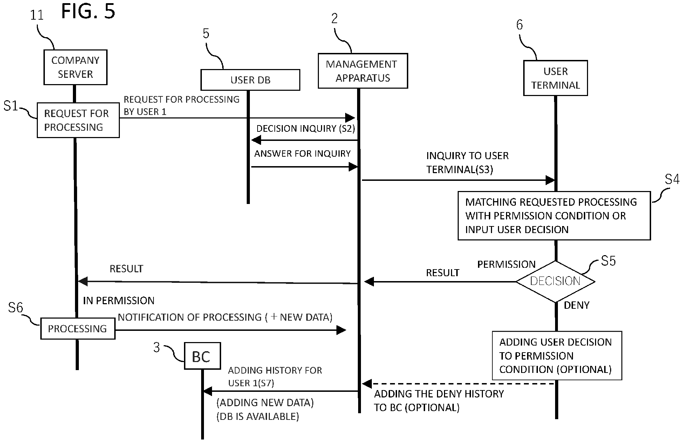

   Fig. 5 illustrates an example of the processing performed for personalized medicine using genomic data obtained from a specimen of a user.
[Fig. 6]

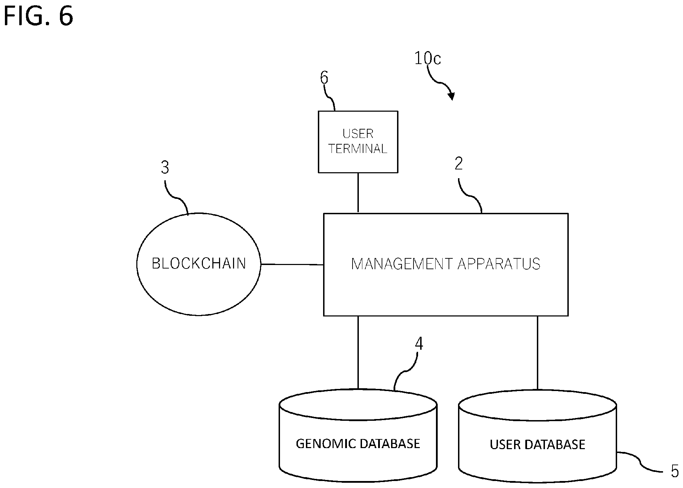

   Fig. 6 is a diagram illustrating an example of the schematic configuration of a genomic data processing system.
[Fig. 7]

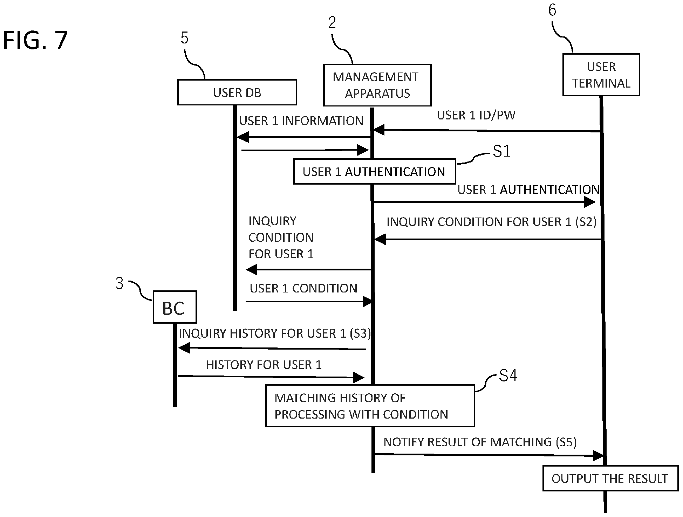

   Fig. 7 illustrates an example of querying the history of processing performed for personalized medicine using genomic data obtained from a specimen of user.
[Fig. 8]

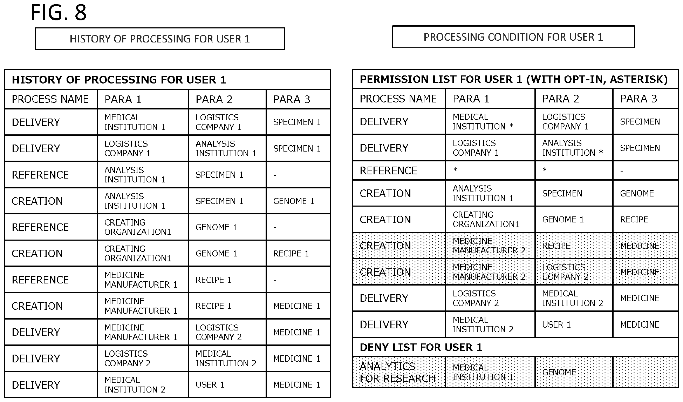

   Fig. 8 illustrates an example of history of processing and permission conditions for a user.
[Fig. 9]

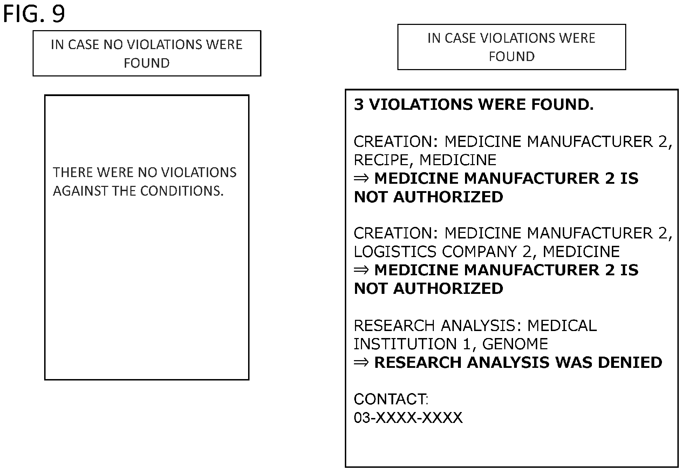

   Fig. 9 illustrates an example of a result of matching processing history with permission conditions.
[Fig. 10]

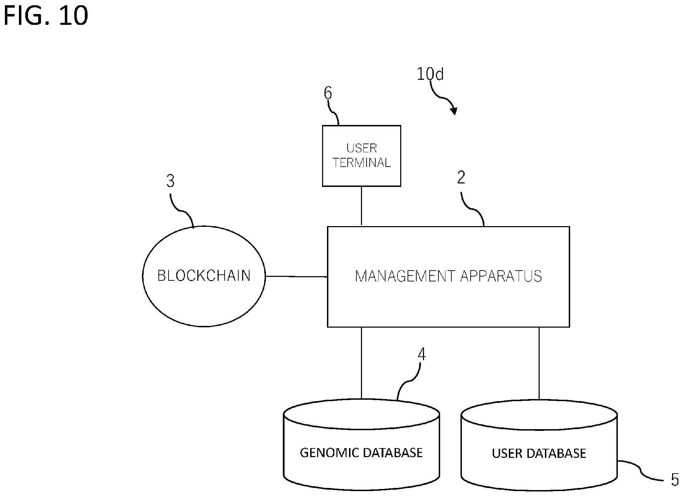

   Fig. 10 is a diagram illustrating an example of the schematic configuration of a genomic data processing system.
[Fig. 11]

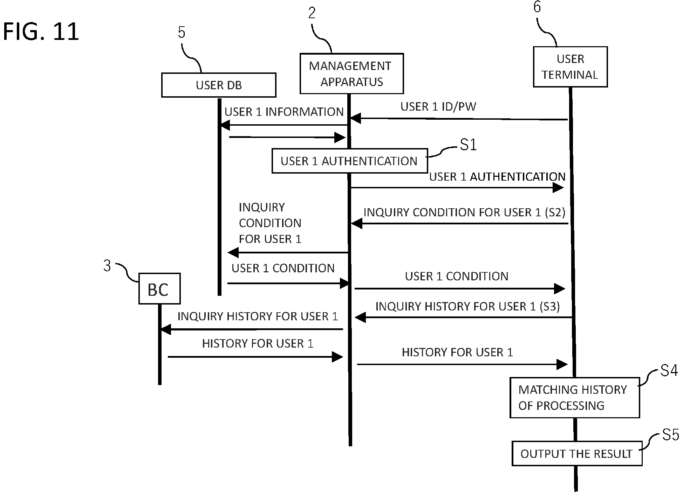

   Fig. 11 illustrates an example of querying the history of processing performed for personalized medicine using genomic data obtained from a specimen of a user.
[Fig. 12]

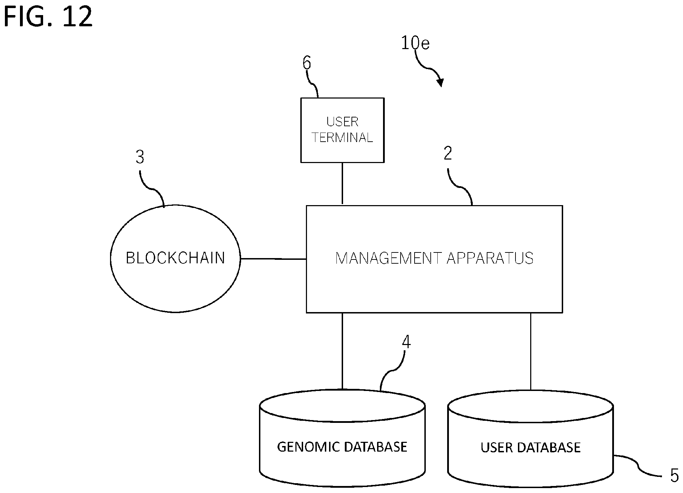

   Fig. 12 is a diagram illustrating an example of the schematic configuration of a genomic data processing system.
[Fig. 13]

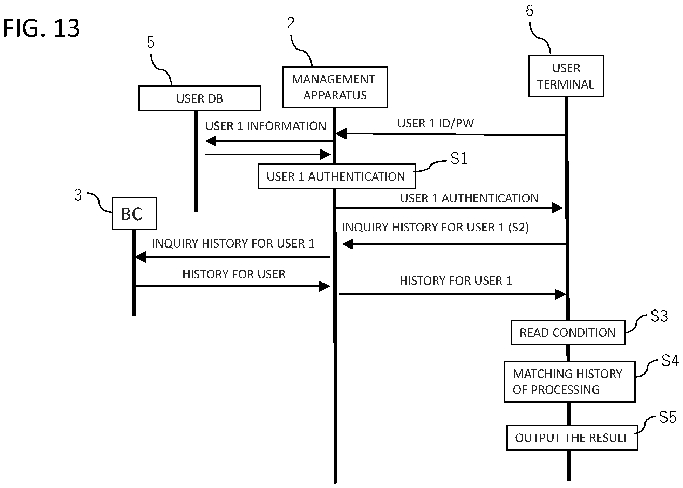

   Fig. 13 illustrates an example of querying the history of processing performed for personalized medicine using genomic data obtained from a specimen of a user.
[Fig. 14]

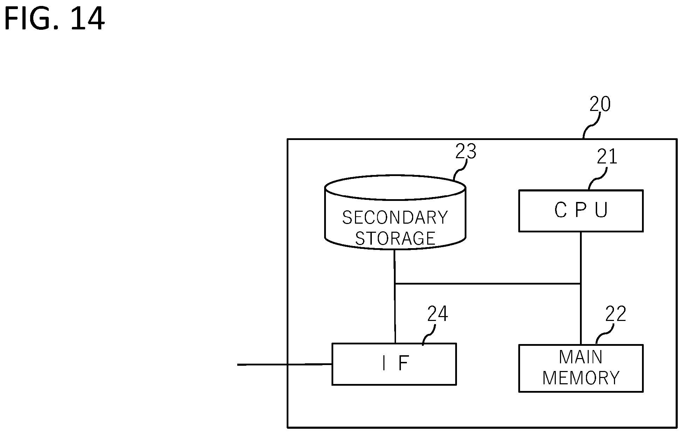

   Fig. 14 is a diagram illustrating an example of the hardware configuration of the company servers and management apparatus used in the embodiments.

### EXAMPLE EMBODIMENTS

The embodiments of the present invention will be described below with reference to the drawings. However, the present invention is not limited to the embodiments described below. Furthermore, in each drawing, identical or corresponding elements are denoted by the same reference numerals. It should also be noted that the drawings are schematic, and the dimensional relationships and proportions of the elements may differ from those in reality. It should also be noted that there may be portions within the drawings where the dimensional relationships or proportions differ from one another.

### (First Embodiment)

Fig. 1 is a diagram illustrating an example of the schematic configuration of a genomic data processing system. As shown in Fig. 1, the genomic data processing system 10a comprises a plurality of company servers 11, 12, and 13, a management apparatus 2, a blockchain 3, a genomic database 4, and a user database 5.

The plurality of company servers 11, 12, and 13 are responsible for performing part of a series of processes for personalized medicine based on genomic data obtained from specimens of a user. Furthermore, the plurality of company servers 11, 12, and 13 submit a request for processing to the management apparatus 2 prior to performing the processing. Additionally, the plurality of company servers 11, 12, and 13 report the results of the processing to the management apparatus 2 after performing the processing. As will be described in detail later, the processing required to perform personalized medicine using genomic data obtained from the specimens of the user consists of a series of consecutive steps. Therefore, while it is conceivable to execute the entire series of processing steps required for personalized medicine using genomic data obtained from the specimens of the user on a single company server, it is also conceivable to distribute the execution among the plural company servers 11, 12, and 13.

Management apparatus 2 manages information regarding a series of processes performed on genomic data. Furthermore, based on permission conditions representing processes authorized by the user, management apparatus 2 notifies multiple company servers 11, 12, and 13 of the approval or rejection result for processes for which the request received. In this configuration, management apparatus 2 stores permission conditions in user database 5 in advance and determines whether to approve or reject the processing of the request received by management apparatus 2. Furthermore, management apparatus 2 registers and records the processing history reported by company servers 11, 12, and 13 on blockchain 3.

Genomic database 4 stores genomic data obtained from the specimen of the user, as well as variant information derived from applying a series of processes to the genomic data. User database 5 stores a list of permission conditions representing the processes authorized by the user. While the genomic data processing system 10a shown in Figure 1 employs a configuration comprising multiple company servers 11, 12, and 13 and a separate management apparatus 2, the management apparatus 2 may alternatively be implemented as part of the functions of the multiple company servers 11, 12, and 13, such that each of the multiple company servers 11, 12, and 13 executes the processing of the management apparatus 2.

Fig. 2 is a schematic diagram illustrating an example of the series of processes applied to genomic data in personalized medicine. As shown in Fig. 2, personalized medicine is typically carried out by multiple companies sharing the responsibilities.

Specimens such as blood and tissue collected from users at medical institutions are sent to sequencing companies. Sequencing companies sequence the received specimens and obtain genomic data (read data) representing the nucleotide sequences of the genomes contained in the specimens. However, since genomic data (read data) is merely the nucleotide sequence of the genome, it cannot be used on its own to provide personalized medicine. Therefore, sequencing companies send the genomic data (read data) to data analysis companies.

Data analysis companies analyze genomic data (read data) to generate variant information that identifies the locations where variants are contained. Here, variant information refers to information regarding variants in the genomic nucleotide sequence, including the locations of nucleotide sequences containing variants and the types of variants, such as single-nucleotide variants, insertions, or deletions. This variant information relates to the individual biological characteristics of a user and is crucial for personalized medicine. However, even with an individual variant information of a user, treatment plans for personalized medicine cannot be determined on their own. Therefore, data analysis companies send genomic information-genomic data annotated with variant information-to ICT (Information and Communication Technology) companies.

ICT companies perform neoantigen analysis using genomic data that includes variant information to determine treatment plans. Neoantigens are cancer antigens (markers) that have newly emerged due to genetic variants in cancer cells, for example, and they provide critical information for determining personalized treatment plans. Furthermore, since these genetic variants do not necessarily occur whenever cancer cells develop, it is necessary to analyze them through neoantigen analysis of genomic data supplemented with variant information. On the other hand, once neoantigen information is obtained, the neoantigens become targets for personalized medicine.

ICT companies send treatment plans derived from neoantigen analysis of genomic data-which includes variant information-to biotechnology companies. Biotechnology companies then manufacture medicines and other products tailored to the treatment plans derived from the neoantigen analysis. The medicines and other products manufactured by biotechnology companies are sent to medical institutions, where they are used to provide personalized medical care to users.

Additionally, the processes described above are examples of a series of procedures applied to genomic data in personalized medicine; in actual practice, different procedures may be performed. For example, neoantigen analysis is one such procedure used in personalized medicine, and in some cases, cancer gene panel testing may be performed instead of neoantigen analysis. Cancer gene panel testing is a test that examines variations in nucleotide sequences to identify medications or clinical trials that are expected to be effective. When an ICT company performs cancer gene panel testing, it sends the information regarding medications or clinical trials expected to be effective-derived from the test results-to a biotechnology company. Biotechnology companies then manufacture medicines and other products suitable for the treatment plans derived from the cancer gene panel testing. These medicines and other products manufactured by the biotechnology companies are sent to medical institutions, where they are used to provide personalized medicine to users.

Thus, in personalized medicine, the process involves not only obtaining genomic data-which consists simply of the nucleotide sequence of the genome-from a specimen, but also performing multiple subsequent processing steps. Consequently, as illustrated in Fig. 1, it is common for a genomic data processing system 10a to comprise multiple company servers 11, 12, and 13, with the processing tasks being distributed and executed across the multiple company servers 11, 12, and 13.

Fig. 3 illustrates an example of the processing performed for personalized medicine using genomic data obtained from a specimen of a user. As shown in Fig. 3, the series of processes for personalized medicine involving genomic data obtained from specimens of a user includes: processing of the request for processing (S1); processing of the condition inquiry (S2); processing to match the request against the permitted conditions (S3); processing to determine whether to permit the processing (S4); processing to actually execute the processing and notify the results (S5); and processing to record the history of the notified processing (S6).

The processing of the request (S1) is intended for company server 11, which performs one of the processes for personalized medicine on genomic data obtained from the specimen of the user, to submit a request for processing to management apparatus 2 prior to processing.

The processing of the condition inquiry (S2) is intended for the management apparatus 2, upon receiving a request for processing, to inquire about the permission conditions representing the processing authorized by the user. Here, it is assumed that the management apparatus 2 holds the permission conditions in the user database 5 in advance and that management apparatus 2 can query the user database 5.

In the processing to match the request against the permitted conditions (S3), the processing requested from company server 11 is matched against the permitted conditions retrieved from the user database 5. In the process of determining whether to approve the request (S4), the management apparatus 2 determines whether to execute the requested process based on the results of comparing the requested process with the permission conditions. If the requested process is approved, the management apparatus 2 sends an approval notification to the company server 11; if it is not approved, the management apparatus 2 sends a rejection notification to the company server 11.

In the process of actually executing the operation and notifying the results (S5), the company server 11, having received the notification of permission, actually executes the processing and notifies the management apparatus 2 of the results. In the process of recording the history of the notified processing (S6), the management apparatus 2, having received the notification of the execution of the processing, registers the processing history in the blockchain 3. Note that the blockchain 3 may also be a conventional database.

Furthermore, if the execution of a process is denied, the management apparatus 2 may send a denial notification to the company server 11 and terminate the process; alternatively, the management apparatus 2 may record the history of the denied process request on the blockchain 3.

Additionally, the information registered in blockchain 3 may include not only information indicating that a process has been executed, but also, for example, a hash value or a combination of hash values derived from a combination of the sample ID, user ID, processing date, processing program name, program version, and processing results (output, evaluation results). While it is preferable to register and record the processing history in the blockchain, it is also possible to include hash values of genomic data.

As described above, in the processing of genomic data obtained from a specimen of a user for personalized medicine, a request for processing must be submitted prior to execution, and the decision to proceed is made based on the permission conditions for a user. This allows the entire sequence of processing for personalized medicine to be managed so that only the processing to which the user has agreed is performed.

### (Second Embodiment)

FIG. 4 is a diagram illustrating an example of the schematic configuration of a genomic data processing system. As shown in FIG. 4, the genomic data processing system 10b comprises multiple company servers 11, 12, and 13; a management apparatus 2; a blockchain 3; a genomic database 4; a user database 5; and a user terminal 6.

Multiple company servers 11, 12, and 13 are responsible for performing part of a series of processes for personalized medicine based on genomic data obtained from a specimen of a user. Furthermore, the multiple company servers 11, 12, and 13 submit a request for processing to the management apparatus 2 prior to performing the processing. Additionally, the multiple company servers 11, 12, and 13 report the results of the processing to the management apparatus 2 after performing the processing. While it is conceivable to perform the series of processes leading up to the implementation of personalized medicine based on genomic data obtained from specimens of a user on a single company server, it is also conceivable to distribute the responsibility among multiple company servers 11, 12, and 13.

Management apparatus 2 manages information regarding a series of processes performed on genomic data. Furthermore, based on permission conditions representing processes authorized by the user, management apparatus 2 notifies multiple company servers 11, 12, and 13 of the result regarding whether to approve or reject a requested process. In this configuration, the user terminal 6 holds the permission conditions in advance and determines the approval or rejection of the processing for which a request has been received. Furthermore, the management apparatus 2 registers and records the history of processing reported by the company servers 11, 12, and 13 on the blockchain 3.

Genomic database 4 stores genomic data obtained from the specimen of a user and mutation information derived from applying a series of processes to that genomic data. User database 5 records the location of a list of permission conditions that represent the processes authorized by the user. In other words, in this configuration, since the user terminal 6 holds the permission conditions, user database 5 records that the user terminal 6 is the target for inquiries regarding whether to permit the execution of a process.

Additionally, although the genomic data processing system 10b shown in Fig. 4 employs a configuration comprising multiple company servers 11, 12, and 13 and a separate management apparatus 2, the management apparatus 2 may be implemented as part of the functions of the multiple company servers 11, 12, and 13, such that each of the multiple company servers 11, 12, and 13 executes the processing of the management apparatus 2.

Fig. 5 illustrates an example of the processing performed for personalized medicine using genomic data obtained from a specimen of a user. As shown in Fig. 5, the series of processes for personalized medicine using genomic data obtained from a specimen of a user comprises: a processing of the request for processing (S1); a process of querying the decision-making entity (S2); a process of querying the user terminal (S3), a process of checking the requested processing against permission conditions (S4), a process of determining whether to permit the processing (S5), a process of actually performing the processing and notifying the result (S6), and a process of recording the history of the notified processing (S7).

The processing of the request for processing (S1) is intended for company server 11, which performs one of the processes for personalized medicine on genomic data obtained from the specimen of a user, to submit a request for processing to management apparatus 2 prior to processing.

In the process of querying the decision-making entity (S2), the management apparatus 2 queries the user database 5 for information regarding the entity authorized to determine whether to approve the requested processing. In this case, the user terminal 6 holds the permission conditions, and the management apparatus 2 obtains the information that it should query the user terminal 6 from the user database 5.

In the process of querying the user terminal (S3), the management apparatus 2 inquires the user terminal 6 as to whether the requested process may be permitted.

In the process of checking the requested processing against permission conditions (S4), user terminal 6 compares the requested processing received from company server 11 with the permission conditions held by user terminal 6 or inputs the decision for the user. In the process of determining whether to permit the processing (S5), user terminal 6 determines whether to permit execution of the requested processing based on the results of comparing the requested processing against the permission conditions. If the execution of the process is permitted, user terminal 6 sends a permission notification to management apparatus 2; if it is not permitted, user terminal 6 sends a denial notification to management apparatus 2. After receiving the notification of the determination result regarding the execution of the process from user terminal 6, management apparatus 2 forwards that determination result to company server 11.

In the process of actually performing the processing and notifying the result (S6), the company server 11 that received the permission notification actually performs the processing and notifies the management apparatus 2 of the result. In the process of recording the history of the notified processing (S7), the management apparatus 2, upon receiving notification of the processing execution, registers the history of the processing on the blockchain 3. Note that the blockchain 3 may also be a conventional database.

If the request is denied, user terminal 6 may send a denial notification to management apparatus 2 and terminate the process; alternatively, management apparatus 2 may record the history of the denied request in blockchain 3.

Additionally, the information registered in blockchain 3 may include not only information indicating that a process has been executed, but also, for example, a hash value or a combination of hash values derived from a combination of the sample ID, user ID, processing date, processing program name, program version, and processing results (output, evaluation results). While it is preferable to register and record the processing history in the blockchain, it is also possible to include hash values of genomic data.

As described above, in the processing of genomic data obtained from a specimen a user for personalized medicine, a request for processing must be submitted prior to execution, and the decision to proceed is made based on the permission conditions a user. This allows the entire sequence of processing for personalized medicine to be managed so that only the processing to which the user has consented is performed.

### (Third Embodiment)

Fig. 6 is a diagram illustrating an example of the schematic configuration of a genomic data processing system. As shown in Fig. 6, the genomic data processing system 10c comprises a management apparatus 2, a blockchain 3, a genomic database 4, a user database 5, and a user terminal 6.

The management apparatus 2 manages information regarding a series of processes performed on genomic data. As described in the first embodiment, the management apparatus 2 registers and records the history of processes reported from the company server onto blockchain 3. Although omitted from the description in this embodiment, the history of the series of processes performed on genomic data within the genomic data processing system is recorded on blockchain 3 using the method described in the first embodiment.

The genomic database 4 stores genomic data obtained from the specimen of a user and variant information derived from applying a series of processes to the genomic data. The user database 5 stores a list of permission conditions representing the processes authorized by the user, as well as the user ID and password used to access the processing history.

Fig. 7 illustrates an example of querying the history of processing performed for personalized medicine using genomic data obtained from a specimen of user. As shown in Fig. 7, the process of querying the history of personalized medicine processing for genomic data obtained from specimens of a user includes user authentication processing (S1), processing to query permission conditions (S2), processing to query the history of processing (S3), processing to match the history of processing against permission conditions (S4), and processing to notify the matching result (S5).

In the user authentication processing (S1), the user terminal 6 transmits the user ID and password to the management apparatus 2. The management apparatus 2 then refers to the user ID and password stored in the user database 5, compares the user ID and password received from the user terminal 6 with those stored in the user database 5, and performs user authentication. The result of the user authentication is notified to the user terminal 6 by the management apparatus 2.

In the process to query permission conditions (S2), user terminal 6 instructs management apparatus 2 to query the permission conditions, and management apparatus 2, upon receiving this instruction, retrieves the permission conditions of a user stored in user database 5. In the processing to query the history of processing (S3), management apparatus 2 retrieves the history of processing related to the user stored in blockchain 3.

In the processing to match the history of processing against permission conditions (S4), the management apparatus 2 compares the history of processing related to the user with the permission conditions of the user. The history of processing related to the user and the permission conditions of the user are stored in a format such as that shown in Fig. 8, and the management apparatus 2 compares the history of processing related to the user with the permission conditions of the user to determine whether only the processing authorized by the user is executed.

In the processing to notify the matching result (S5), the management apparatus 2 notifies the user terminal 6 of the results of matching the history of processing regarding a user with the permission conditions for the user. As shown in Fig. 9, for example, if only the processing authorized by the user is executed, the notification displays a message indicating that there is no violation of conditions; if processing other than that authorized by the user is executed, the notification displays a list of unauthorized processing.

In the genomic data processing system 10c and the genomic data processing method in the third embodiment described above, as in the first embodiment, a request for processing is submitted before execution, and the permissibility of processing is determined by comparing it against the permission conditions representing the processing authorized by the user. Consequently, it ensures that only processing to which the user has consented is performed, and since the user can view the history of executed processing, the series of processes for personalized medicine can be managed even more strictly.

### (Fourth Embodiment)

Fig. 10 is a diagram illustrating an example of the schematic configuration of a genomic data processing system. As shown in Fig. 10, the genomic data processing system 10d comprises a management apparatus 2, a blockchain 3, a genomic database 4, a user database 5, and a user terminal 6.

Management apparatus 2 manages information regarding the series of processes performed on genomic data. As described in the first embodiment, management apparatus 2 registers and records the history of processes reported from the company server onto blockchain 3. Although omitted from the description in this embodiment, the history of the series of processes performed on genomic data within the genomic data processing system is recorded on blockchain 3 using the method described in the first embodiment.

The genomic database 4 stores genomic data obtained from the specimen of a user and variant information derived from applying a series of processes to the genomic data. The user database 5 stores a list of permission conditions representing the processes authorized by the user, as well as the user ID and password used to access the history of the processes.

Fig. 11 illustrates an example of querying the history of processing performed for personalized medicine using genomic data obtained from a specimen of a user. As shown in Fig. 11, the process of querying the history of processing performed for personalized medicine using genomic data obtained from a specimen of a user includes processing for user authentication (S1), querying permission conditions (S2), processing for querying the history of processing (S3), processing for matching the history of processing against permission conditions (S4), and processing for outputting the results of the matching (S5).

In the user authentication processing (S1), the user terminal 6 transmits the user ID and password to the management apparatus 2. The management apparatus 2 then refers to the user ID and password stored in the user database 5, compares the user ID and password received from the user terminal 6 with those stored in the user database 5, and performs user authentication. The result of the user authentication is notified to the user terminal 6 by the management apparatus 2.

In the process to query permission conditions (S2), user terminal 6 instructs management apparatus 2 to query the permission conditions, and management apparatus 2, upon receiving this instruction, retrieves the permission conditions for a user stored in user database 5 and submits the permission conditions to the user terminal 6. In the processing to query the history of processing (S3), management apparatus 2 retrieves the history of processing related to the user stored in blockchain 3.

In the processing to match the history of processing against permission conditions (S4), the management apparatus 2 compares the history of processing related to the user with the permission conditions of the user. The history of processing related to the user and the permission conditions of the user are stored in a format such as that shown in Fig. 8, and the management apparatus 2 compares the history of processing related to the user with the permission conditions of the user to determine whether only the processing authorized by the user is executed.

In the process for outputting the results of the matching (S5), the results of matching the history of processing regarding a user against the permission conditions for the user are displayed on the user terminal 6. As shown in Fig. 9, for example, if only the processing authorized by the user has executed, a message indicating that there is no violation of conditions is displayed; if processing other than that authorized by the user has executed, a list of unauthorized processing is displayed.

In the genomic data processing system 10d and the genomic data processing method in the fourth embodiment described above, as in the first embodiment, a request for processing is submitted before execution, and the permissibility of processing is determined by comparing it against the permission conditions representing the processing authorized by the user. Consequently, it ensures that only processing to which the user has consented is performed, and since the user can view the history of executed processing, the series of processes for personalized medicine can be managed even more strictly.

### (Fifth Embodiment)

Fig. 12 is a diagram illustrating an example of the schematic configuration of a genomic data processing system. As shown in Fig. 12, the genomic data processing system 10e comprises a management apparatus 2, a blockchain 3, a genomic database 4, a user database 5, and a user terminal 6.

Management apparatus 2 manages information regarding the series of processes performed on genomic data. As described in the first embodiment, management apparatus 2 registers and records the history of processes reported from the company server onto blockchain 3. Although omitted from the description in this embodiment, the history of the series of processes performed on genomic data within the genomic data processing system is recorded on blockchain 3 using the method described in the first embodiment.

The genomic database 4 stores genomic data obtained from the specimen of a user and variant information derived from applying a series of processes to that genomic data. The user database 5 records the location of a list of permission conditions that represent the processes authorized by the user. In other words, in this configuration, the user terminal 6 holds the permission conditions, and the user database 5 records that the user terminal 6 is the entity to which inquiries regarding whether to permit processing.

Fig. 13 illustrates an example of querying the history of processing performed for personalized medicine using genomic data obtained from a specimen of a user. As shown in Fig. 13, the process of querying the history of personalized medicine processing performed on genomic data obtained from specimens of a user includes processing for user authentication (S1), processing for querying the history of processing (S2), processing for retrieving permission conditions (S3), processing for matching the history of processing against the permission conditions (S4), and processing for outputting the results of the match (S5).

In the process for user authentication (S1), the user terminal 6 transmits the user ID and password to the management apparatus 2. The management apparatus 2 then references the user ID and password stored in the user database 5, compares the user ID and password received from the user terminal 6 with those stored in the user database 5, and performs user authentication. The result of the user authentication is notified to the user terminal 6 by the management apparatus 2.

In the processing for querying the history of processing (S2), the user terminal 6 instructs the management apparatus 2 to query the history of processing, and the management apparatus 2 retrieves the history of processing related to the user stored in the blockchain 3 and transmits it to the user terminal 6. In the processing for retrieving the permission conditions (S3), the permission conditions recorded in the user terminal 6 are retrieved.

In the processing to match the history of processing against permission conditions (S4), the management apparatus 2 compares the history of processing related to the user with the permission conditions of the user. The history of processing related to the user and the permission conditions of the user are stored in a format such as that shown in Fig. 8, and the management apparatus 2 compares the history of processing related to the user with the permission conditions of the user to determine whether only the processing authorized by the user is executed.

In the process for outputting the results of the matching (S5), the results of matching the history of processing regarding a user against the permission conditions for the user are displayed on the user terminal 6. As shown in Fig. 9, for example, if only the processing authorized by the user has executed, a message indicating that there is no violation of conditions is displayed; if processing other than that authorized by the user has executed, a list of unauthorized processing is displayed.

In the genomic data processing system 10e and the genomic data processing method in the fifth embodiment described above, as in the second embodiment, a request for processing is submitted before execution, and the permissibility of processing is determined by comparing it against the permission conditions representing the processing authorized by the user. Consequently, it ensures that only processing to which the user has consented is performed, and since the user can view the history of executed processing, the series of processes for personalized medicine can be managed even more strictly.

### (Example Hardware Configuration)

Fig. 14 is a diagram illustrating an example of the hardware configuration of the company servers and management apparatus used in the embodiments. Specifically, company servers 11, 12, and 13, and management apparatus 2 enable the realization of their respective functions by performing the genomic data processing method described above as a program on an information processing apparatus (computer) 20 that adopts the hardware configuration shown in Fig. 14. However, the hardware configuration example shown in Fig. 14 is merely one example of a hardware configuration for realizing the respective functions of company servers 11, 12, 13, and management apparatus 2, and is not intended to limit the hardware configuration of company servers 11, 12, 13, and management apparatus 2. Company servers 11, 12, 13, and management apparatus 2 may include hardware not shown in Fig. 4.

As shown in Fig. 14, the hardware configuration that company servers 11, 12, and 13 and management apparatus 2 may adopt includes, for example, a CPU (Central Processing Unit) 21, main memory 22, secondary storage 23, and an interface (IF) unit 24, all interconnected via an internal bus.

The CPU 21 executes each instruction contained in the genomic data processing program executed by the information processing apparatus (computer) 20. The main memory 22 is, for example, RAM (Random Access Memory), and temporarily stores various programs, such as the genomic data processing program executed by the information processing apparatus (computer) 20, so that the CPU 21 can process them.

The secondary storage device 23 is, for example, an HDD (hard disk drive), and is capable of storing various programs, such as the genomic data processing program executed by the information processing apparatus (computer) 20, for the medium to long term. Various programs, such as the genomic data processing program, can be provided as program products recorded on a non-transitory computer-readable storage medium.

IF unit 24 provides interfaces for the input/output of, for example, company servers 11, 12, and 13, and management apparatus 2.

The information processing apparatus (computer) 20, which employs the hardware configuration described above, implements the functions of company servers 11, 12, and 13 and management apparatus 2 by executing the aforementioned genome information processing method as a program.

Part or all of the above embodiments may be described as set forth in the following notes, but are not limited thereto.

### [Supplementary Note 1]

A genomic data processing system comprising:
a company server responsible for a part of a series of processes for personalized medicine using genomic data obtained from a specimen of a user, and
a management apparatus that manages information regarding the series of processes performed on said genomic data, wherein
the company server submits a request for processing to the management apparatus prior to executing the processing, and
the management apparatus notifies the company server of a result regarding whether the requested processing is permitted or not, based on permission conditions representing a processing authorized by the user.

### [Supplementary Note 2]

The genomic data processing system according to Supplementary Note 1, wherein the management apparatus stores the permission conditions and determines whether to permit processing of the request received by the management apparatus.

### [Supplementary Note 3]

The genomic data processing system according to Supplementary Note 1, wherein a terminal of the user stores the permission conditions, and the management apparatus determines whether to permit the processing in the request by querying the user terminal.

### [Supplementary Note 4]

The genomic data processing system according to any one of Supplementary Notes 1 to 3, wherein
the company server reports a result of a processing to the management apparatus after performing the processing, and
the management apparatus registers and records a history of the reported processing on a blockchain.

### [Supplementary Note 5]

The genomic data processing system according to Supplementary Note 4, wherein the management apparatus registers and records the result of permissibility of the processing for which the request was received on the blockchain.

### [Supplementary Note 6]

The genomic data processing system according to Supplementary Note 4 or Supplementary Note 5, wherein the management apparatus stores the permission conditions in advance, and, upon receiving a request from the terminal of the user regarding the history of the processing, compares the history of the processing with the permission conditions and notifies the terminal of the user of a result of the comparison.

### [Supplementary Note 7]

The genomic data processing system according to Supplementary Note 4 or Supplementary Note 5, wherein the management apparatus stores the permission conditions in advance, and, upon receiving a request from the terminal of the user regarding the history of the processing, compares the history of the processing with the permission conditions and notifies the terminal of the user of the result of the comparison.

### [Supplementary Note 8]

A method for processing genomic data, using a company server responsible for part of a series of processes for personalized medicine based on genomic data obtained from a specimen of a user, and a management apparatus that manages information regarding the series of processes performed on said genomic data, wherein
the company server submits a request for processing to the management apparatus prior to executing the processing, and
the management apparatus notifies the company server of a result regarding whether the requested processing is permitted or not, based on permission conditions representing a processing authorized by the user.

### [Supplementary Note 9]

The genomic data processing method according to Supplementary Note 8, wherein
the company server reports processing to the management apparatus after executing the processing, and
the management apparatus registers and records the history of the reported processing on a blockchain.

### [Supplementary Note 10]

A genomic data processing program that causes a company server responsible for part of a series of processes for personalized medicine based on genomic data obtained from a specimen of a user, and a management apparatus that manages information regarding the series of processes performed on said genomic data, to execute genomic data processing, wherein the genomic data processing program causes
the company server submit a request for processing to the management apparatus prior to executing the processing, and
the management apparatus notify the company server of a result regarding whether the requested processing is permitted or not, based on permission conditions representing a processing authorized by the user.

Further, the disclosure of Patent Literature cited above is incorporated herein in its entirety by reference thereto. It is to be noted that it is possible to modify or adjust the example embodiments or examples within the scope of the whole disclosure of the present invention (including the Claims) and based on the basic technical concept thereof. Further, it is possible to variously combine or select (or partially omit) a wide variety of the disclosed elements (including the individual elements of the individual claims, the individual elements of the individual example embodiments or examples, and the individual elements of the individual figures) within the scope of the whole disclosure of the present invention. That is, it is self-explanatory that the present invention includes any types of variations and modifications to be done by a skilled person according to the whole disclosure including the Claims and the technical concept of the present invention. Particularly, any numerical ranges disclosed herein should be interpreted that any intermediate values or subranges falling within the disclosed ranges are also concretely disclosed even without specific recital thereof. In addition, using some or all of the disclosed matters in the literatures cited above as necessary, in combination with the matters described herein, as part of the disclosure of the present invention in accordance with the object thereof shall be considered to be included in the disclosed matters of the present application.

### REFERENCE SIGNS LIST

10a, 10b, 10c, 10d, 10e: genome information processing systems
11, 12, 13: company servers
2: management apparatus
3: blockchain
4: genomic database
5: user database
6:user terminal
20: information processing apparatus (computer)
21: CPU (Central Processing Unit)
22: main memory
23: secondary storage
24: IF (interface) unit

## Claims

1. A genomic data processing system comprising:
a company server responsible for a part of a series of processes for personalized medicine using genomic data obtained from a specimen of a user, and
a management apparatus that manages information regarding the series of processes performed on said genomic data, wherein
the company server submits a request for processing to the management apparatus prior to executing the processing, and
the management apparatus notifies the company server of a result regarding whether the requested processing is permitted or not, based on permission conditions representing a processing authorized by the user.

2. The genomic data processing system according to claim 1, wherein the management apparatus stores the permission conditions and determines whether to permit processing of the request received by the management apparatus.

3. The genomic data processing system according to claim 1, wherein a terminal of the user stores the permission conditions, and the management apparatus determines whether to permit the processing in the request by querying the user terminal.

4. The genomic data processing system according to any one of claims 1 to 3, wherein
the company server reports a result of the processing to the management apparatus after performing the processing, and
the management apparatus registers and records a history of the reported processing on a blockchain.

5. The genomic data processing system according to claim 4, wherein the management apparatus registers and records the result of permissibility of the processing for which the request was received on the blockchain.

6. The genomic data processing system according to claim 4 or claim 5, wherein the management apparatus stores the permission conditions in advance, and, upon receiving a request from the terminal of the user regarding the history of the processing, compares the history of the processing with the permission conditions and notifies the terminal of the user of a result of the comparison.

7. The genomic data processing system according to claim 4 or claim 5, wherein the terminal of the user stores the permission conditions, when the management apparatus receives a request from the user terminal regarding the history of the processing, the management apparatus notifies the terminal of the user of the history of the processing, and the terminal of the user compares the history of the processing with the permission conditions.

8. A method for processing genomic data, using a company server responsible for part of a series of processes for personalized medicine based on genomic data obtained from a specimen of a user, and a management apparatus that manages information regarding the series of processes performed on said genomic data, wherein
the company server submits a request for processing to the management apparatus prior to executing the processing, and
the management apparatus notifies the company server of a result regarding whether the requested processing is permitted or not, based on permission conditions representing a processing authorized by the user.

9. The genomic data processing method according to claim 8, wherein
the company server reports processing to the management apparatus after executing the processing, and
the management apparatus registers and records the history of the reported processing on a blockchain.

10. A genomic data processing program that causes a company server responsible for part of a series of processes for personalized medicine based on genomic data obtained from a specimen of a user, and a management apparatus that manages information regarding the series of processes performed on said genomic data, to execute genomic data processing, wherein the genomic data processing program causes
the company server submit a request for processing to the management apparatus prior to executing the processing, and
the management apparatus notify the company server of a result regarding whether the requested processing is permitted or not, based on permission conditions representing a processing authorized by the user.
